# EUROPEAN PATENT SPECIFICATION

(11) **EP 2 848 260 B1**
(45) Date of publication and mention of the grant of the patent: **23.03.2016**
(21) Application number: 14184430.8
(22) Date of filing: 11.09.2014
(51) Int. Cl.: A61K 47/44, A61K 9/20, A61K 31/16

(54) **Novel formulations of thiocolchicoside**
Neuartige Formulierungen von Thiocolchicosid
Nouvelles formulations de thiocolchicoside

(30) Priority: 12.09.2013 TR 201310731; 12.09.2013 TR 201310746
(43) Date of publication of application: 18.03.2015
(73) Proprietor: Sanovel Ilac Sanayi ve Ticaret A.S., 34460 Istanbul (TR)
(72) Inventor: Türkyilmaz, Ali, 34460 Istanbul (TR); Pehlivan Akalin, Nur, 34460 Istanbul (TR)
(74) Representative: Sevinç, Erkan

(56) References cited:
- WO-A1-2011/112161
- WO-A1-2012/144964
- WO-A2-2010/103544

## Description

### Technical field

Present invention relates to novel pharmaceutical formulations comprising an effective amount of thiocolchicoside or pharmaceutically acceptable salts thereof for oral administration with myorelaxant activity.

### Background of the Invention

Muscle relaxants are used in the management of musculoskeletal and neuromuscular disorders. They also reduce muscle tone and are used in therapy for the treatment of muscle spasm and contractures.

Muscle spasm is one of the main factors responsible for chronic pain; it characterises several pathologies of the locomotor apparatus as well as inflammatory-rheumatic and degenerative orthopaedic pathologies. When it affects joints, they cause not only pain, but also rigidity, which reduces joint mobility and flexibility in the affected part. Muscle contractures also characterize several pathologies of the locomotor apparatus and are one of the main factors responsible for the persistence of the pain associated to these pathologies.

For these reasons, the study of molecules endowed with muscle relaxant and antispasmodic properties still raises remarkable interest from the clinical point of view.

As it is known, colchicine is a pseudoalcaloid that has been widely used for a long time in therapy for the treatment of gout. The use of 3- demethyl- thiocolchicine glucoside, known as thiocolchicoside, is also widespread in therapy for treating contractures and inflammatory conditions that affect the muscular system.

Thiocolchicoside has been claimed to possess GABA-mimetic and glycinergic actions. In other words we can say that thiocolchicoside is a gamma-aminobutiric acid receptor agonist and it is used in the treatment of painful muscle spasms or spasticity occurring in musculoskeletal and neuromuscular disorders and for treating contractures and inflammatory conditions that affect the muscular system. Its chemical structure is shown in Formula 1.

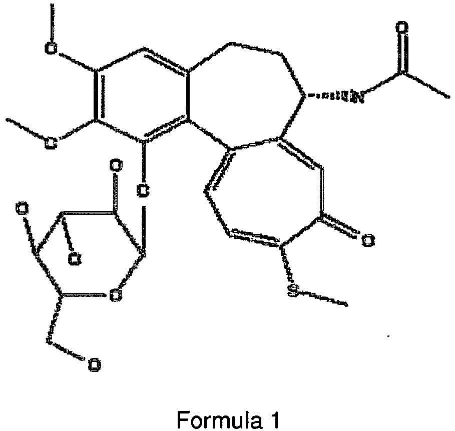

The usual initial dose is 16 mg daily by mouth. It has also been given intramuscularly, in doses up to 8 mg daily, or applied as cream or ointment.

It is known in the prior art that, thiocolchicoside is a highly soluble active ingredient and almost completely absorbed after oral administration. It seems to be advantegous for the cases that require immediate action for the treatment. However, because of the mentioned properties of thiocolchicoside, immediate release formulations of thiocolchicoside are required to be administered three-times daily or four times daily to maintain the therapeutic effect over an extended period of time and this results in fluctuations of drug concentration in the blood plasma and undergoes repeated chemical imbalances, which might be detrimental to the patient's health and lead to undesired side effects. Three-times daily or four times daily dosing regimens of the conventional immediate release formulations is very inconvenient and leads to reduction in patient compliance.

Additionally, it is also known that the tablet hardness also affects the relase of the active agent in such a manner that increase of the tablet hardness causes the decrease of the release of the active agent. Since the hardness of the tablets increased with the increased compression force, the compression force is a control parameter to adjust the hardness of the tablet obtained. Increase of the tablet hardness can result in decrease of the release of the active agent, on the other hand it can improve the physical properties of the tablet. Therefore, it is important to adjust the hardness of the sustained release tablet carefully by applying the sufficient compression force to provide desired release of active agent in a controlled manner over extended period of time as well as to improve the physical properties of it.

The various formulations of thiocolchicoside are described in prior art as follows:
EP 1052995B1 discloses a pharmaceutical composition containing thiocolchicoside which is in the form of a powder and is administered by nasal administration.
WO1998036751A1 discloses a solid pharmaceutical composition of thiocolchicoside for administration via the buccal mucosa in the absorption site.
WO 1996/041635A1 discloses a pharmaceutical composition containing a diclofenac salt and thiocolchicoside.

Taking consideration of the prior art, there is still a need for a pharmaceutical formulation of thiocolchicoside which comes over aforementioned disadvantages and providing additional advantages over the art.

Accordingly the object of the invention is to develop a pharmaceutical formulation of thiocolchicoside that provides once daily or twice daily dosing for effective management of pain, spasticity, inflammatory symptoms and painful muscle spasms, an improved side effect and increased patient compliance.

### Description of the figures

**Figure 1** shows a bilayer tablet formulation of the invention which (a) represents immediate release phase of thiocolchicoside, and (b) represents sustained release phase of thiocolchicoside.
**Figure 2** shows a multilayer tablet comprising a barrier layer in which (c) represents barrier layer between immediate release phase and sustained release phase.
**Figure 3** shows a tablet formulation of the invention in which (e) represents the core comprising the sustained released thioocolchicoside, and (d) represents the coating layer comprising the drug which is released immediately.
**Figure 4** shows a multicoated tablet formulation of the invention in which (h) represents a core containing sustained-release thiocolchicoside and pharmaceutically acceptable excipients, and (f) represents a polymer barrier coating layer, (g) a coating layer comprising thiocolchicoside which is released immediately.

### Objects of the Invention

The object of the present invention is to provide a pharmaceutical formulation of thiocolchicoside comprising immediate release phase, and sustained release phase comprising glyceryl behenate as rate controlling polymer, for the treatment of pain, spasticity, inflammatory symptoms and painful muscle spasms by eliminating all the aforementioned drawbacks and providing additional advantages to the respective technical field.

Another object of the present invention is to provide a pharmaceutical formulation making the plasma concentration level stable by maintaining release of thiocolchicoside in the blood stream for a longer time period sufficient to justify once daily or twice daily dosing.

Another object of the present invention is to provide a pharmaceutical formulation maintaining the therapeutic effect due to the sustained release of thiocolchicoside over extended period of time that increases patient compliance.

Another object of the present invention is to provide a pharmaceutical formulation that shows a release profile of thiocolchicoside regardless of tablet hardness and compression force.

Another object of the present invention is to provide a pharmaceutical formulation that can be produced with good physical properties to be resistant to capping, aberration or breakage under conditions of storage, transportation and handling before usage.

Another object of the present invention is to provide a process for the manufacture of said pharmaceutical formulation of thiocolchicoside.

### Detailed description of the invention

This invention is a novel formulation of thiocolchicoside for oral administration with antiinflammatory, analgesic, myorelaxant activity and methods of its manufacture.

The term "sustained release phase" refers to any pharmaceutical formulation that maintain constant levels of a drug in the patient's bloodstream by releasing the drug over an extended period of time. Sustained release phase are formulated to release the active ingredient gradually and predictably over a 12-hour to 24-hour period.

The term "immediate release phase" refers to any pharmaceutical formulations that disintegrate rapidly after administration with enhanced rate of dissolution and get dissolved to release the medicaments.

The term "rate controlling polymer" refers to an excipient in the final dosage form whose primary function is to modify the duration of release of the active drug substance from the dosage form.

The term "dosage form" refers to any form of the formulation that contains the active agent(s) in an amount sufficient to achieve a therapeutic effect with a single administration.

The term "active agent" refers to the agent, ingredient, drug or other substance that provides some beneficial pharmacological effect for the treatment. Within the scope of the invention, the active agent is thiocolchicoside or a pharmaceutically acceptable salt thereof.

The term "pharmaceutically acceptable excipient" refers to any ingredient having no therapeutic activity and being nontoxic and thus suitable as an excipient.

The present invention relates to a pharmaceutical formulation of thiocolchicoside comprising immediate release phase, and sustained release phase comprising glyceryl behenate as rate controlling polymer.

Thiocolchicoside is a highly and rapidly soluble active ingredient and almost completely absorbed after oral administration. It is known that, it is difficult to develop a sustained release pharmaceutical formulations of highly and rapidly soluble pharmaceuticals. It is also hard to achieve the desired dissolution profiles in other words the control of the release rate of the active agent is difficult. Therefore, fluctuation of the active agent concentration in the blood plasma may occur which may lead to toxicity. Also, diurnal variation of the active ingredient in plasma is also possible.

The present invention relates to a pharmaceutical formulation of thiocolchicoside comprising immediate release phase, and sustained release phase comprising glyceryl behenate as rate controlling polymer that provides such therapeutic relief by releasing thiocolchicoside in such a manner that dose dumping is prevented and requisite blood levels are maintained for an extended time period sufficient to justify once daily or twice daily dosing and thus increase patient compliance.

According to present invention, the pharmaceutical formulation of thiocolchicoside comprising immediate release phase, and sustained release phase comprising glyceryl behenate as rate controlling polymer, wherein each of phases comprising thiocolchicoside or a pharmaceutically acceptable salt thereof.

According to the present invention, the pharmaceutical formulation releases immediately thiocolchicoside to provide sufficient blood concentration in a short time for starting therapeutic effect by means of immediate release phase and it provides release of thiocolchicoside for a long duration by means of sustained release phase to maintain required effective blood concentration for the therapeutic effect. Therefore, the sustained release pharmaceutical formulation makes the plasma concentration level stable by maintaining release of thiocolchicoside in the blood stream for a longer time period sufficient to justify once daily or twice daily dosing and this increases patient compliance. Also, reduction of a dose regimen enables fluctuations of the active ingredient concentration in the blood plasma to be prevented.

According to the present invention, the pharmaceutical formulation is administered orally in tablet, bilayer tablet, multilayer tablet or multicoated tablet form.

Immediate release phase of said formulation provide rapid release of thiocolchicoside after administration to provide sufficient blood concentration in a short time for starting therapeutic effect. Sustained release of thiocolchicoside is achieved by the sustained release phase of said formulation to make the plasma concentration level stable by maintaining release of thiocolchicoside in the blood stream for a longer time period. Therefore, the release profile of thiocolchicoside in sustained release phase is very important to maintain the therapeutic effect. As above mentioned, the hardness of the tablet has an important role for the release of the active agent, i.e. thiocolchicoside in the invention. Increase of the hardness of the tablet affects adversely the release of the active agent in the formulation and thus causes the release of the active agent to be decreased. Additionally, the hardness of the tablet is effected form magnitude of the compression force applied to form the tablet in such manner that the hardness of the tablet increases when the compression force increases. Additionally, the hardness of the tablet also affects significantly the physical properties of the tablet that are important for the resistance of the tablet obtained.

Suprisingly, it has been found that when glyceryl behenate is used as rate controlling polymer in the formulation of the invention, the release of thiocolchicoside is independent of the hardness of the tablet. In other words, the release of thiocolchicoside from sustained release phase of the formulation is regardless of the compression forced applied to form the tablet. Therefore, whatever the magnitude of the applied compression force is, the release profile of thiocolchicoside is almost stable even if the hardness of the tablet changes.

Therefore, use of glyceryl behenate as rate controlling polymer enables the sustained release pharmaceutical formulation of thiocolchicoside of the present invention to be more advantageous over the prior art. Use of glyceryl behenate enables the sustained release pharmaceutical formulation to be successful in achieving the desired release profil of thiocolchicoside even if the hardness of the tablet is substantially high or substantially low. Therefore, the dissolution rate of thiocolchicoside, which is associated with the hardness of the tablet in such a manner that the dissolution rate of thiocolchicoside decreases with increase of the hardness of the tablet, is also unaffected from the hardness of the formulation by means of glyceryl behenate content of the formulation. Thus, it is also possible to obtain the tablet with desired dissolution rate even if the hardness of the tablet is substantially high or low.

Additionally, use of glyceryl behenate enables the release of thiocolchicoside to be regardless of the tablet hardness, therefore it makes also possible to produce tablets with optimal hardness that enables the tablets to be produced with good physical properties to be resistant to capping, aberration, seperation or breakage under conditions of storage, transportation and handling before usage.

One aspect of the present invention, the percent amount of glyceryl behenate in said sustained release phase is between 1% to 50%, preferably 5% to 40%, more preferably between 10% to 30%, most preferably between 15% to 25% by weight based on the total weight of the sustained release phase.

Another aspect of the present invention, said pharmaceutical formulation may contain additional rate controlling polymer selected from a group comprising glyceryl palmitostearte, glyceryl monostearate, polyglycolized glycerides hydrogenated vegetable oils such as hydrogenated castor oil, microcrystalline wax, hydroxypropylmethylcellulose, hydroxypropylcellulose, cetyl alcohol, a polymethacrylate or mixtures thereof.

Another aspect of the invention, the pharmaceutical formulation further comprise a polymethacrylate, that is also identified as ammonio methacrylate copolymer, as a rate controlling polymer.

According the present invention, ammonio methacrylate copolymer is preferably in aqueous dispersion form with low permeability and this aqueous dispersion contains a polymer dry weight content preferably between 10% and 50%, more preferably between between 25% to 50%, most preferably of 30% (available under tradename Eudragit RS 30 D) based on the total weight of the aqueous dispersion.

During production of the pharmaceutical formulation according to the present invention, firstly, ammonio methacrylate copolymer is added in aqueous dispersion form. Howewer, after drying step of the production, water content of the aqueous dispersion is substantially lost, and the polymer dry weight content is remained in the final sustained release formulation. Therefore, the polymer dry weight content of ammonio methacrylate copolymer remained in the final formulation is between 0.5% to 15%, preferably between 1% to 12%, more preferably between 2% to 9% by weight based on the total weight of final the formulation.

It is found that when the ratio of the total weight of ammonio methacrylate copolymer aqueous dispersion to the total weight of glyceryl behenate is between 1:0.1 and 1:30 in the sustained release phase, ammonio methacrylate copolymer helps glyceryl behenate to maintains the release of thiocolchicoside over a more extended period of time. The ratio of the total weight of ammonio methacrylate copolymer aqueous dispersion to the total weight of glyceryl behenate is preferably between 1:0.5 and 1:20, more preferably between 1:1 and 1:10 in the sustained release phase.

According to present invention, the active agent is thiocolchicoside or a pharmaceutically acceptable salts thereof. The pharmaceutical formulation, preferably in tablet, bilayer tablet, multilayer tablet or multicoated tablet form, contains immediate release phase and sustained release phase. %1-25% of thiocolchicoside content of the pharmaceutical formulation is in the immediate release phase, the remaining 75%-99% of thiocolchicoside content of said formulation is in the sustained release phase.

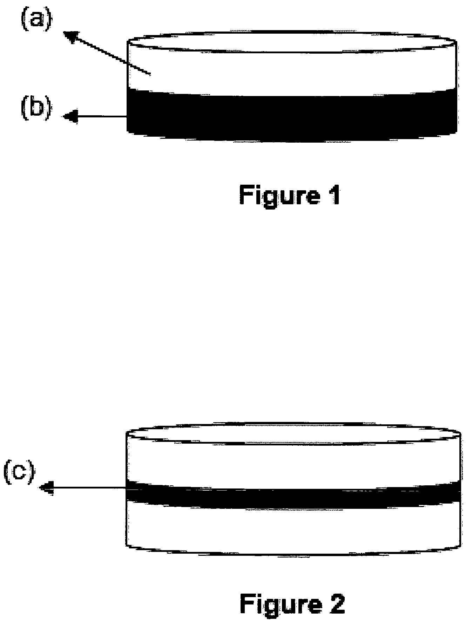

One of the aspect of the present invention, a pharmaceutical formulation of thiocolchicoside comprising immediate release phase, and sustained release phase comprising glyceryl behenate as rate controlling polymer *(**Figure 1**)*.

Another aspect of the present invention, another phase such as a barrier layer is used in the middle of the multilayer tablet in order to obtain a separation between both phases easily, without damaging the surface areas *(**Figure 2**)*. When this multilayer tablet contacts with the dissolution media they separate and act like two independent tablets. Additionally, by means of barrier layer, the probable interactions between different formulations can be minimized.

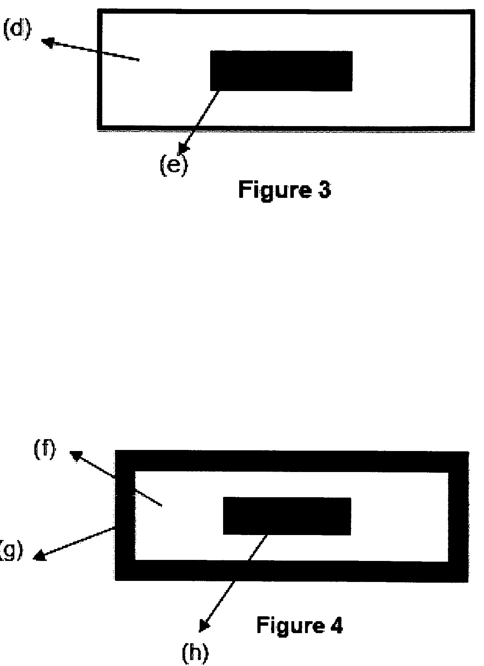

Another aspect of the present invention, the pharmaceutical composition is in the form of a bilayer tablet comprising:
a. a core comprising the sustained released thiocolchicoside and pharmaceutically acceptable excipients *(**Figure 3 (e)**)*,
b. a coating layer comprising thiocolchicoside and pharmaceutically acceptable excipients wherein thiocolchicoside is released immediately *(**Figure 3 (d)**)*.

Another aspect of the present invention, the pharmaceutical formulation is in the form of a multicoated tablet comprising:
a. a core comprising sustained-release thiocolchicoside and pharmaceutically acceptable excipients *(**Figure 4(h)**)*,
b. a polymer coating barrier layer *(**Figure 4(f)**),*
c. a coating layer comprising thiocolchicoside and pharmaceutically acceptable excipients wherein thiocolchicoside is released immediately *(**Figure 4(g)**)*.

Total weight of thiocolchicoside or a pharmaceutically acceptable salts thereof in the formulation of the invention is between 2 mg and 20 mg, preferably between 8 mg and 16 mg.

Pharmaceutically formulations will typically be prepared in admixture with pharmaceutical acceptable excipients. Suitable excipients include, but are not limited to: water; salt solutions; alcohols; gum arabic; vegetable oils; benzyl alcohol; polyethylene glycols; gelatin; carbohydrates such as lactose, amylose or starch; magnesium stearate; talc; silicic acid; paraffin; perfume oil; fatty acid esters; hydroxypropylmethylcellulose; polyvinyl pyrrolidone; etc.

According to the present invention, the pharmaceutical formulation can be sterilized, and can be in an oral dosage forms include tablets, multilayer tablets (coated or uncoated), multicoated tablets.

Another aspect of the present invention, immediate release phase and sustained release phase of the pharmaceutical formulation further comprises at least one pharmaceutically acceptable excipient selected from a group comprising lubricants, binding agents, fillers, preservatives, disintegrants, plasticizers, aromatic substances, or a mixture thereof. Suitable fillers are selected from the group comprising starch, lactose (lactose monohydrate), microcrystalline cellulose (e.g. Avicel® PH 101), carboxy cellulose sodium, sucrose; suitable binding agents are selected from the group comprising povidone, hydroxypropylcellulose, hydroxypropylmethylcellulose, ethylcellulose, starch (e.g. corn starch), gelatin (e.g. Gelatin 200 Bloom), sugar; suitable lubricants are selected from the group comprising collodial anhydrous silica, magnesium stearate, talc, sodium stearyl fumarate; suitable disintegrants are selected from the group comprising microcrystalline cellulose, sodium starch glycollate, croscarmellose sodium, crospovidone, starch and their mixtures; suitable plasticizer are selected from the group comprising diethyl phthalate (DEP), triethyl citrate; suitable glidants are selected from the group comprising colloidal silicon dioxide, silicon dioxide, magnesium trisilicate, powdered cellulose, starch, talc, tribasic calcium phosphate, lactose, stearates, dibasic calcium phosphate, magnesium carbonate, magnesium oxide, calcium silicate, silicon dioxide aerogels and their mixtures; suitable aromatic agents are selected from the group comprising fruit aromas such as of orange, cherry, strawberry, banana, sourcherry, lemon, etc.; aromas of cardamom, anis, mint, menthol, eucalyptus, vanillin, and ethyl vanillin, and the mixtures thereof; suitable preservatives are selected from the group comprising methylparaben and propylparaben and the salts thereof (e.g. sodium, potassium), sodium benzoate, citric acid, benzoic acid, butylated hydroxytoluene and butylated hydroxyanisole, and the mixtures thereof.

According to the present invention, the pharmaceutical formulation has an immediate release phase comprising thiocolchicoside and it preferably comprises;
a. 0.1% to 15.0% by weight of thiocolchicoside,
b. 25.0% to 75.0% by weight of lactose monohydrate,
c. 5.0% to 30.0 % corn starch,
d. 0.5% to 5.0 % by weight of gelatin,
e. 0.5% to 5.0 % by weight of sugar,
f. 0.5% to 10.0 % by weight of talc,
g. 0.5% to 10.0 % by weight of magnesium stearate.

According to the present invention, the pharmaceutical formulation has a sustained release phase comprising thiocolchicoside and it preferably comprises;
a. 5.0% to 20.0% by weight of thiocolchicoside,
b. 10.0% to 30.0% by weight of glyceryl behenate,
c. 10.0% to 20.0% by weight of ammonio methacrylate copolymer aqueous dispersion,
d. 25.0% to 70.0% by weight of microcrystalline cellulose,
e. 0.5% to 5.0% by weight of talc,
f. 1.0% to 5.0% by weight of sodium stearyl fumarate.

The ammonio methacrylate copolymer aqueous dispersion comprised by abovementioned sustained release phase of the formulation contains a polymer dry weight content preferably of 30% (available under tradename Eudragit RS 30 D) based on the total weight of the aqueous dispersion.

The present invention concern the use of the pharmaceutical formulations comprising thiocolchicoside for the manufacture of a medicament for the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

According to the invention, the pharmaceutical formulation of thiocolchicoside is preferably in the form of a tablet, multilayer tablet or multicoated tablet. According to the present invention it may be produced by any standard tabletting technique, e.g. by wet granulation, dry granulation or direct compression.

According to another aspect, preferred process for the manufacture of the pharmaceutical formulations of the invention comprises the following steps:
a) for the preparation of immediate release phase:
   i. thiocolchicoside, lactose monohydrate, corn starch, sugar are mixed to form a mixture,
   ii. aqueous solution of gelatin is prepared, and then it is added onto dry mixture prepared in step i) for granulation,
   iii. wet granules are grinded, and then are dried to obtain them with the moisture content of maximum 3.0%,
   iv. dry granules ontained in step iii) passed through grinder and transferred to a mixer to mix with talc,
   v. magnesium stearate is added to the mixture obtained in step iv) and they are mixed.
b) for preparation of sustained release phase:
   i) thiocolchicoside, glyceryl behenate and microcrystalline cellulose are mixed to form a mixture,
   ii) obtained mixture is granulated with ammonio methacrylate copolymer aqueous dispersion,
   iii) granules are sieved, then dried in a drying oven at the temperature of 40°C-60°C until having moisture content of 2% and milled,
   iv) talc is mixed with the granules, then sodium stearil fumarate is added and mixed with granules.

The homogeneous mixtures obtained in step a) and b) are compressed to obtain a tablet. Optionally, the tablets are preferably covered by a coating material including conventional coating polymers like Opadry®.

This invention is further defined by reference to the following examples. Although the examples are not intended to limit the scope of the present invention, it should be considered in the light of the description detailed above.

### Example:

### Example 1:

The pharmaceutical formulation is consisting of sustained release phase and immediate release phase wherein each of them comprises thiocolchicoside and pharmaceutically acceptable excipients. The formulations of immediate release phase and sustained release phase given below:

| ***Content of immediate release phase*** | ***% amount (w*/*w)*** |
|---|---|
| Thiocolchicoside | 1.0 - 10.0 |
| Lactose monohydrate | 55.0 - 75.0 |
| Corn starch | 10.0 - 20.0 |
| Gelatin | 0.5 - 3.0 |
| Sugar | 0.5 - 3.0 |
| Talc | 1.0 - 6.0 |
| Magnesium stearate | 1.0 - 6.0 |
| **Total weight of immediate release phase** | **34 mg** |

| ***Content of sustained release phase*** | ***% amount (w*/*w)*** |
|---|---|
| Thiocolchicoside | 8.0 - 15.0 |
| Glyceryl behenate | 15.0 - 27.5 |
| Ammonio methacrylate copolymer aqueous dispersion* | 10.0 - 17.5 |
| Microcrystalline cellulose | 40.0 - 60.0 |
| Talc | 1.0 - 2.5 |
| Sodium stearyl fumarate | 1.0 - 4.0 |
| **Total weight of sustained release phase** | **126 mg** |

| | |
|---|---|
| ** with a polymer dry content of 30% based on the total weight of the aqueous dispersion (Eudragit RS 30 D)* | |

The immediate release phase of the pharmaceutical formulation is prepared as follows: thiocolchicoside, lactose monohydrate, corn starch, sugar are mixed to form a mixture, aqueous solution of gelatin is prepared, and then it is added onto dry mixture prepared in step i) for granulation, wet granules are grinded, and then are dried to obtain them with the moisture content of maximum 3.0%, dry granules ontained in step iii) passed through grinder and transferred to a mixer to mix with talc for 5 minutes, magnesium stearate is added to the mixture obtained in step iv) and they are mixed for 3 minutes.

The sustained release phase of the pharmaceutical formulation is prepared as follows: thiocolchicoside, glyceryl behenate and microcrystalline cellulose are mixed to form a mixture; obtained mixture is granulated with ammonio methacrylate copolymer aqueous dispersion with a polymer dry weight content of 30%; granules are sieved, then dried in a drying oven at the temperature of 40°C-60°C until having moisture content of 2% and milled until having a particle size of 700 µm; talc is mixed with the granules for 5 minutes, then sodium stearil fumarate is added and mixed with granules for 3 minutes.

The homogeneous mixtures obtained in the preparation of the immediate release phase and sustained release phase are compressed to obtain a tablet *(**Figure* 1). Optionally, the tablets are preferably covered by a coating material including conventional coating polymers like Opadry®.

### Example 2:

| ***Content of immediate release phase*** | ***% amount (w*/*w)*** |
|---|---|
| Thiocolchicoside | 1.0 - 10.0 |
| Lactose monohydrate | 55.0 - 75.0 |
| Corn starch | 10.0 - 20.0 |
| Gelatin | 0.5 - 3.0 |
| Sugar | 0.5 - 3.0 |
| Talc | 1.0 - 6.0 |
| Magnesium stearate | 1.0 - 6.0 |
| **Total weight of immediate release phase** | **34 mg** |

| ***Content of barrier layer*** | ***% amount (w*/*w)*** |
|---|---|
| Microcrystalline cellulose | 70.0 - 99.0 |
| Hydroxypropyl cellulose | 0.1 - 15.0 |
| Iron oxide yellow | 0.05 - 5.0 |
| Colloidal silicon dioxide | 0.05 - 5.0 |
| Magnesium stearate | 0.1 - 5.0 |
| **Total weight of barrier layer** | **75 mg** |

| ***Content of sustained release phase*** | ***% amount (w*/*w)*** |
|---|---|
| Thiocolchicoside | 8.0 - 15.0 |
| Glyceryl behenate | 15.0 - 27.5 |
| Ammonio methacrylate copolymer aqueous dispersion* | 10.0 - 17.5 |
| Microcrystalline cellulose | 40.0 - 60.0 |
| Talc | 1.0 - 2.5 |
| Sodium stearyl fumarate | 1.0 - 4.0 |
| **Total weight of sustained release phase** | **126 mg** |

| | |
|---|---|
| ** with a polymer dry content of 30% based on the total weight of the aqueous dispersion (Eudragit RS 30 D)* | |

The immediate release phase of the pharmaceutical formulation is prepared as follows: thiocolchicoside, lactose monohydrate, corn starch, sugar are mixed to form a mixture, aqueous solution of gelatin is prepared, and then it is added onto dry mixture prepared in step i) for granulation, wet granules are grinded, and then are dried to obtain them with the moisture content of maximum 3.0%, dry granules ontained in step iii) passed through grinder and transferred to a mixer to mix with talc for 5 minutes, magnesium stearate is added to the mixture obtained in step iv) and they are mixed for 3 minutes.

Barrier layer of the pharmaceutical formulation is prepared as follows: a part of microcrystalline cellulose and hydroxypropyl cellulose, that are previously weighed, are taken into an intermediate bulk container. Iron oxide yellow, colloidal silicon dioxide and the remaining part of microcrystalline cellulose are passed through a sieve with the pore size of 630 µm and then taken into the intermediate bulk container. The powder mixture is mixed for 20 minutes with a SP 2000 Blender. Magnesium stearate is added to this mixture and then they are mixed for 5 minutes.

The sustained release phase of the pharmaceutical formulation is prepared as follows: thiocolchicoside, glyceryl behenate and microcrystalline cellulose are mixed to form a mixture; obtained mixture is granulated with ammonio methacrylate copolymer aqueous dispersion with a polymer dry weight content of 30%; granules are sieved, then dried in a drying oven at the temperature of 40°C-60°C until having moisture content of 2% and milled until having a particle size of 700 µm; talc is mixed with the granules for 5 minutes, then sodium stearil fumarate is added and mixed with granules for 3 minutes.

The homogeneous mixtures obtained in the preparation of the immediate release phase, barrier layer and sustained release phase are compressed to obtain a tablet (Figure 2). Optionally, the tablets are preferably covered by a coating material including conventional coating polymers like Opadry®.

## Claims

1. A pharmaceutical formulation of thiocolchicoside comprising immediate release phase, and a sustained release phase comprising glyceryl behenate as rate controlling polymer.

2. The pharmaceutical formulation according to claim 1, wherein the amount of glyceryl behenate is between 1% to 50% by weight based on the total weight of the sustained release phase.

3. The pharmaceutical formulation according to claim 2, wherein the amount of glyceryl behenate is between 5% to 40% by weight based on the total weight of the sustained release phase.

4. The pharmaceutical formulation according to claim 3, wherein the amount of glyceryl behenate is between 10% to 30% by weight based on the total weight of the sustained release phase.

5. The pharmaceutical formulation according to any of preceding claims further comprising additional rate controlling polymer selected from a group comprising glyceryl palmitostearte, glyceryl monostearate, polyglycolized glycerides, hydrogenated vegetable oils such as hydrogenated castor oil, microcrystalline wax, cetyl alcohol, a polymethacrylate or mixtures thereof.

6. The pharmaceutical formulation according to claim 5, wherein the additional rate controlling polymer is a polymethacrylate.

7. The pharmaceutical formulation according to claim 6, wherein the polymethacrylate is ammonio methacrylate copolymer.

8. The pharmaceutical formulation according to claim 7, wherein ammonio methacrylate copolymer is in aqueous dispersion form.

9. The pharmaceutical formulation according to any of preceding claims, wherein the ratio of the total weight of ammonio methacrylate copolymer aqueous dispersion to the total weight of glyceryl behenate is between 1:0.1 and 1:30 in the sustained release phase.

10. The pharmaceutical formulation according to claim 9, wherein the ratio of the total weight of ammonio methacrylate copolymer aqueous dispersion to the total weight of glyceryl behenate preferably between 1:0.5 and 1:20 in the sustained release phase.

11. The pharmaceutical formulation according to claim 10, wherein wherein the ratio of the total weight of ammonio methacrylate copolymer aqueous dispersion to the total weight of glyceryl behenate is preferably between 1:1 and 1:10 in the sustained release phase.

12. The pharmaceutical formulation according to any of preceding claims, wherein said pharmaceutical formulation is in the form of a tablet, a bilayer tablet, a multilayer tablet or a multicoated tablet.

13. The pharmaceutical formulation according to any of preceding claims, wherein %1-25% of thiocolchicoside content of the pharmaceutical formulation is in the immediate release phase, the remaining 75%-99% of thiocolchicoside is in the sustained release phase.

14. The pharmaceutical formulation according to any of preceding claims comprises:
- an immediate release phase comprising:
a. 0.1% to 15.0% by weight of thiocolchicoside,
b. 25.0% to 75.0% by weight of lactose monohydrate,
c. 5.0% to 30.0 % corn starch,
d. 0.5% to 5.0 % by weight of gelatin,
e. 0.5% to 5.0 % by weight of sugar,
f. 0.5% to 10.0 % by weight of talc,
g. 0.5% to 10.0 % by weight of magnesium stearate; and
- a sustained release phase comprising:
a. 5.0% to 20.0% by weight of thiocolchicoside,
b. 10.0% to 30.0% by weight of glyceryl behenate,
c. 10.0% to 20.0% by weight of ammonio methacrylate copolymer aqueous dispersion,
d. 25.0% to 70.0% by weight of microcrystalline cellulose,
e. 0.5% to 5.0% by weight of talc,
f. 1.0% to 5.0% by weight of sodium stearyl fumarate.

15. A process for the manufacture of the pharmaceutical formulations according to any of preceding claims comprises the following steps:
a) for the preparation of the immediate release phase:
i. thiocolchicoside, lactose monohydrate, corn starch, sugar are mixed to form a mixture,
ii. aqueous solution of gelatin is prepared, and then it is added onto dry mixture prepared in step i) for granulation,
iii. wet granules are grinded, and then are dried to obtain them with the moisture content of maximum 3.0%,
iv. dry granules ontained in step iii) passed through grinder and transferred to a mixer to mix with talc,
v. magnesium stearate is added to the mixture obtained in step iv) and they are mixed;
b) for preparation of sustained release phase:
i. thiocolchicoside, glyceryl behenate and microcrystalline cellulose are mixed to form a mixture,
ii. obtained mixture is granulated with ammonio methacrylate copolymer aqueous dispersion,
iii. granules are sieved, then dried in a drying oven at the temperature of 40°C-60°C until having moisture content of 2% and milled,
iv. talc is mixed with the granules, then sodium stearil fumarate is added and mixed with granules,
and the homogeneous mixtures obtained in step a) and b) are compressed to obtain a tablet.

## Patentansprüche

1. Pharmazeutische Formulierung von Thiocolchicosid umfassend eine schnell freisetzende Phase und eine retardiert freisetzende Phase umfassend Glycerinbehenat als geschwindigkeitskontrollierendes Polymer.

2. Pharmazeutische Formulierung nach Anspruch 1, wobei die Menge an Glycerinbehenat zwischen 1% bis 50% bezogen auf das Gewicht basierend auf dem Gesamtgewicht der retardiert freisetzenden Phase beträgt.

3. Pharmazeutische Formulierung nach Anspruch 2, wobei die Menge an Glycerinbehenat zwischen 5% bis 40% bezogen auf das Gewicht basierend auf dem Gesamtgewicht der retardiert freisetzenden Phase beträgt.

4. Pharmazeutische Formulierung nach Anspruch 3, wobei die Menge an Glycerinbehenat zwischen 10% bis 30% bezogen auf das Gewicht basierend auf dem Gesamtgewicht der retardiert freisetzenden Phase beträgt.

5. Pharmazeutische Formulierung nach einem beliebigen der voranstehenden Ansprüche, weiterhin umfassend ein zusätzliches geschwindigkeitskontrollierendes Polymer ausgewählt aus einer Gruppe umfassend Glycerinpalmitostearat, Glycerinmonostearat, polyglycolisierte Glyceride, hydrierte Pflanzenöle wie hydriertes Rizinusöl, mikrokristallines Wachs, Cetylalkohol, ein Polymethacrylat, oder Gemische davon.

6. Pharmazeutische Formulierung nach Anspruch 5, wobei das zusätzliche geschwindigkeitskontrollierende Polymer ein Polymethacrylat ist.

7. Pharmazeutische Formulierung nach Anspruch 6, wobei das Polymethacrylat Ammonio-Methacrylat-Copolymer ist.

8. Pharmazeutische Formulierung nach Anspruch 7, wobei Ammonio-Methacrylat-Copolymer in wässriger Dispersionsform vorliegt.

9. Pharmazeutische Formulierung nach einem beliebigen der voranstehenden Ansprüche, wobei das Verhältnis des Gesamtgewichts von wässriger Dispersion von Ammonio-Methacrylat-Copolymer zum Gesamtgewicht an Glycerinbehenat zwischen 1:0,1 und 1:30 in der retardiert freisetzenden Phase beträgt.

10. Pharmazeutische Formulierung nach Anspruch 9, wobei das Verhältnis des Gesamtgewichts von wässriger Dispersion von Ammonio-Methacrylat-Copolymer zum Gesamtgewicht an Glycerinbehenat bevorzugt zwischen 1:0,5 und 1:20 in der retardiert freisetzenden Phase beträgt.

11. Pharmazeutische Formulierung nach Anspruch 10, wobei das Verhältnis des Gesamtgewichts von wässriger Dispersion von Ammonio-Methacrylat-Copolymer zum Gesamtgewicht an Glycerinbehenat bevorzugt zwischen 1:1 und 1:10 in der retardiert freisetzenden Phase beträgt.

12. Pharmazeutische Formulierung nach einem beliebigen der voranstehenden Ansprüche, wobei die pharmazeutische Fomulierung in der Form einer Tablette, einer Doppelschicht-Tablette, einer Mehrschicht-Tablette, oder einer mehrfach beschichteten Tablette ist.

13. Pharmazeutische Formulierung nach einem beliebigen der voranstehenden Ansprüche, wobei sich 1%-25% des Thiocolchicosidgehalts der pharmazeutischen Fomulierung in der schnell freisetzenden Phase befinden, die verbleibenden 75%-99% befinden sich in der retardiert freisetzenden Phase.

14. Pharmazeutische Formulierung nach einem beliebigen der voranstehenden Ansprüche umfasst:
- eine schnell freisetzende Phase umfassend:
a) 0,1% bis 15,0% bezogen auf das Gewicht Thiocolchicosid,
b) 25,0% bis 75,0% bezogen auf das Gewicht Lactosemonohydrat,
c) 5,0% bis 30,0% Maisstärke,
d) 0,5% bis 5,0% bezogen auf das Gewicht Gelatine,
e) 0,5% bis 5,0% bezogen auf das Gewicht Zucker,
f) 5% bis 10,0% bezogen auf das Gewicht Talk,
g) 0,5% bis 10,0% bezogen auf das Gewicht Magnesiumstearat; und
- eine retardiert freisetzende Phase umfassend:
a. 5,0% bis 20,0% bezogen auf das Gewicht Thiocolchicosid,
b. 10,0% bis 30,0% bezogen auf das Gewicht Glycerinbehenat,
c. 10,0% bis 20,0% bezogen auf das Gewicht wässrige Dispersion von Ammonio-Methacrylat-Copolymer,
d. 25,0% bis 70,0% bezogen auf das Gewicht mikrokristalline Cellulose,
e. 0,5% bis 5,0% bezogen auf das Gewicht Talk,
f.1,0% bis 5,0% bezogen auf das Gewicht Natriumstearylfumarat.

15. Verfahren zur Herstellung der pharmazeutischen Formulierungen nach einem beliebigen der voranstehenden Ansprüche umfasst die folgenden Schritte:
a) zur Herstellung der schnell freisetzenden Phase:
i. Thiocolchicosid, Lactosemonohydrat, Maisstärke, Zucker werden gemischt, um ein Gemisch zu ergeben,
ii. wässrige Lösung von Gelatine wird hergestellt, und wird dann auf das in Schritt i) hergestellte trockene Gemisch zum Granulieren zugegeben,
iii. nasse Körnchen werden gemahlen und dann getrocknet, um sie mit dem Feuchtigkeitsgehalt von maximal 3,0% zu erhalten,
iv. in Schritt iii) erhaltene trockene Körnchen werden durch ein Mahlwerk gelassen und dann in einen Mischer überführt, um mit Talk gemischt zu werden,
v. Magnesiumstearat wird zum in Schritt iv) erhaltenen Gemisch zugegeben, und sie werden gemischt;
b) zur Herstellung der retardiert freisetzenden Phase:
i. Thiocolchicosid, Glycerinbehenat und mikrokristalline Cellulose werden gemischt, um ein Gemisch zu bilden,
ii. das erhaltene Gemisch wird mit einer wässrigen Dispersion von Ammonio-Methacrylat-Copolymer granuliert,
iii. Körnchen werden gesiebt und dann in einem Trockenofen bei der Temperatur von 40°C bis 60°C getrocknet, bis sie einen Feuchtigkeitsgehalt von 2% aufweisen, und gemahlen,
iv. Talk wird mir den Körnchen gemischt, dann wird Natriumstearylfumarat zugegeben und mit den Körnchen gemischt,
und die in Schritt a) und b) erhaltenen homogenen Gemische werden komprimiert, um eine Tablette zu erhalten.

## Revendications

1. Une formulation pharmaceutique de thiocolchicoside comprenant une phase à libération immédiate et une phase à libération prolongée comprenant du béhénate de glycéryle en tant polymère de contrôle de vitesse de libération.

2. La formulation pharmaceutique selon la revendication 1, dans laquelle la quantité de béhénate de glycéryle est comprise entre 1 et 50% en poids rapporté au poids total de la phase à libération prolongée.

3. La formulation pharmaceutique selon la revendication 2, dans laquelle la quantité de béhénate de glycéryle est comprise en 5 et 40% en poids rapporté au poids total de la phase à libération prolongée.

4. La formulation pharmaceutique selon la revendication 3, dans laquelle la quantité de béhénate de glycéryle est comprise entre 10 et 30% en poids rapporté au poids total de la phase à libération prolongée.

5. La formulation pharmaceutique selon l'une quelconque des revendications précédentes, comprenant en outre un polymère supplémentaire de contrôle de vitesse de libération choisi dans le groupe comprenant le palmitostéarate de glycéryle, le monostéarate de glycéryle, les glycérides polyglycolisés, les huiles végétales hydrogénées telles que l'huile de ricin hydrogénée, une cire microcristalline, l'alcool cétylique, un polyméthacrylate ou des mélanges de ces composés.

6. La formulation pharmaceutique selon la revendication 5, dans laquelle le polymère supplémentaire de contrôle de vitesse de libération est un polyméthacrylate.

7. La formulation pharmaceutique selon la revendication 6, dans laquelle le polyméthacrylate est un copolymère de méthacrylate d'ammonium.

8. La formulation pharmaceutique selon la revendication 7, dans laquelle le copolymère de méthacrylate d'ammonium est sous forme de dispersion aqueuse.

9. La formulation pharmaceutique selon l'une quelconque des revendications précédentes, dans laquelle le rapport du poids total de la dispersion aqueuse de copolymère de méthacrylate d'ammonium sur le poids total du béhénate de glycéryle est compris entre 1 :0,1 et 1 :30 dans la phase à libération prolongée.

10. La formulation pharmaceutique selon la revendication 9, dans laquelle le rapport du poids total de la dispersion aqueuse de copolymère de méthacrylate d'ammonium sur le poids total du béhénate de glycéryle est de préférence compris entre 1 :0,5 et 1 :20 dans la phase à libération prolongée.

11. La formulation pharmaceutique selon la revendication 10, dans laquelle le rapport du poids total de la dispersion aqueuse de copolymère de méthacrylate d'ammonium sur le poids total du béhénate de glycéryle est de préférence compris entre 1 :1 et 1 :10 dans la phase à libération prolongée.

12. La formulation pharmaceutique selon l'une quelconque des revendications précédentes, dans laquelle ladite formulation pharmaceutique est sous la forme d'un comprimé, d'un comprimé bicouche, d'un comprimé multicouche ou d'un comprimé à enrobage multiple.

13. La formulation pharmaceutique selon l'une quelconque des revendications précédentes, dans laquelle de 1 à 25% de la teneur de thiocolchicoside de la formulation pharmaceutique se trouve dans la phase à libération immédiate, le reste, 99 à 75% du thicolchicoside, étant dans la phase à libération prolongée.

14. La formulation pharmaceutique selon l'une quelconque des revendications précédentes, comprenant :
- une phase à libération immédiate comprenant :
a. 0,1 à 15,0% en poids de thiocolchicoside,
b. 25,0 à 75,0% en poids de monohydrate de lactose,
c. 5,0 à 30,0% d'amidon de mais,
d. 0,5 à 5,0% en poids de gélatine,
e. 0,5 à 5,0% en poids de sucre,
f. 0,5 à 10,0% en poids de talc,
g. 0,5% à 10,0% en poids de stéarate de magnésium ; et
- une phase à libération prolongée comprenant :
a. 5,0 à 20,0% en poids de thiocolchicoside,
b. 10,0 à 30,0% en poids de béhénate de glycéryle,
c. 10,0 à 20,0% en poids de dispersion aqueuse de copolymère de méthacrylate d'ammonium,
d. 25,0 à 70,0% en poids de cellulose microcristalline,
e. 0,5 à 5,0% en poids de talc,
f. 1,0 à 5,0% en poids de fumarate de stéaryl-sodium.

15. Un procédé de préparation de formulations pharmaceutiques selon l'une quelconque des revendications précédentes, comprenant les étapes suivantes :
a) pour la préparation de la phase à libération immédiate :
i. du thiocolchicoside, du monohydrate de lactose, de l'amidon de maïs, du sucre sont mélangés pour former un mélange,
ii. une solution aqueuse de gélatine est préparée, puis elle est ajoutée dans le mélange sec préparé à l'étape i) pour réaliser une granulation,
iii. les granulés humides sont broyés, ensuite ils sont séchés afin d'obtenir une teneur en humidité d'au maximum 3,0%,
iv. les granulés secs obtenus à l'étape iii) sont fait passer à travers un broyeur et transférés à un mélangeur pour les mélanger à du talc,
v. du stéarate de magnésium est ajouté au mélange obtenu à l'étape iv) et ensuite ils ont mélangés ;
b) pour la préparation de la phase à libération prolongée :
i. du thiocolchicoside, du béhénate de glycéryle et de la cellulose microcristalline sont mélangés pour former un mélange,
ii. le mélange obtenu est granulé avec une dispersion aqueuse de copolymère de méthacrylate d'ammonium,
iii. les granulés sont tamisés, puis séchés dans une étuve à une température de 40 à 60°C jusqu'à ce qu'ils aient une teneur en humidité de 2%, puis broyés,
iv. du talc est mélangé avec les granulés et ensuite du fumarate de stéaryl-sodium est ajouté et mélangé avec les granulés,
et les mélanges homogènes obtenus aux étapes a) et b) sont comprimés pour obtenir un comprimé.
